# EUROPEAN PATENT APPLICATION

(11) **EP 2 811 022 A1**
(43) Date of publication of application: **10.12.2014**
(21) Application number: 14157378.2
(22) Date of filing: 28.02.2014
(51) Int. Cl.: C12N 15/10

(54) **Method for isolating nucleic acids from formalin-fixed paraffin embedded tissue samples**

(30) Priority: 06.06.2013 TW 102120065
(71) Applicant: RBC Bioscience Corp., New Taipei City 23145 (TW)
(72) Inventor: Chung, Ting-Hao, 404 Taichung City (TW); Kuan, Cheng-Chun, 231 New Taipei CIty (TW); Kuo, Shih-Yu, TW / 231 New Taipei City (TW)
(74) Representative: Lang, Christian

(57) **Abstract**

Methods are disclosed for isolating nucleic acids from formalin-fixed paraffin embedded (FFPE) tissue samples. Each of tissue samples contains paraffin and a target biological tissue or material, and the method includes the steps of: adding a first reagent and a second reagent to the FFPE tissue sample, the first reagent dissolving the paraffin material and the second reagent lysing the biological tissue; mixing the first reagent, the second reagent, and the FFPE tissue sample to form a first mixture; (2) heating the first mixture at 50-80°C for 30-90 minutes; and then heating the first mixture at 80-95°C for 30-90 minutes to fractionize the first mixture to form an aqueous phase and an oil phase; (3) collecting an aqueous solution from the aqueous phase; and (4) isolating nucleic acids from the aqueous solution. The method improves the efficiency and convenience of isolating nucleic acids from FFPE tissue samples.

## Description

This application claims priority from Taiwan Patent Application No. 102120065, filed June 6, 2013, the contents of which are hereby incorporated by reference in their entirety for all purposes.

### FIELD OF THE INVENTION

The invention relates to the field of isolation of nucleic acids, particularly, to methods of isolating nucleic acids from formalin-fixed paraffin-embedded biological tissue samples.

### BACKGROUND OF THE INVENTION

Preserving biological samples in the way of formalin-fixed paraffin-embedded tissue specimens is the most widely used method for tissue biopsy. Formalin-fixed paraffin-embedding technique is an essential tool for pathological diagnosis and research, thus the formalin-fixed paraffin-embedded (FFPE) specimens are invaluable resource of biological samples from medical procedures.

After being removed from the patient and being archived, the biological tissues are formalin-fixed and paraffin-embedded for future reference, which solves the problem of tissue decomposition. FFPE specimens are well preserved in view of tissue morphology for the long-term reference. Moreover, FFPE specimens are ideal for archiving and constructing pathological database, and thus the well-archived FFPE specimens and corresponding pathological database provide researchers valuable resources to track back for reference to adequately acquire detail pathological information of the patient at any time.

Generally, FFPE specimens are more commonly used in pathological section for various histochemical or immune-histochemical staining methods followed by microscopy evaluation of diagnostic purpose. In this way, the tissue morphology and specific target biological molecules such as proteins, nucleic acid, bone tissue, etc. are clearly shown for obtaining huge amounts of information for precise diagnosis or pathological investigation. In addition to the aforementioned informations, FFPE specimens also preserve other materials, namely nucleic acids, which are rich in valuable genetic and epigenetic informations. A variety of biomolecular and immunological assays can be optionally conducted after obtaining the nucleic acids according to the purpose, for example, the polymerase chain reaction (PCR), the reverse transcription-PCR (RT-PCR), quantitative real time-PCR (Q-RT-PCR), microarray, immuno-precipitation and so on. Via those analysis based on the nucleic acids, the genetic profile of the FFPE specimens can be further approached, and it significantly contributes to go deep into the mechanism of pathology of various diseases.

On the other hand, practically, as the genomic science is vigorously advanced, a research field named pharmacogenomics becomes mature. In brief, lots of efforts are made to elucidate how genes affect a person's response to drugs, and a slight genetic difference between individuals may result in distinct clinical outcomes in response to drugs. Hence, applying the unique materials of individuals, namely the nucleic acids, to study drug effects and mechanisms is crucial for evaluating the potential response of individual patient to drugs, which is helpful to prescribe or to make other clinical decisions. In other words, utilizing the materials contained within the FFPE specimens can further provide the genetic profile of individual patient for a more precise diagnosis.

Obviously, FFPE specimens not only play critical roles in conventional pathological analysis, which provides valuable resources for long-term preservation, but also systematically provides abundant genetic information, which becomes an ideal tool for medical research. Therefore, to properly isolate intact nucleic acids from FFPE specimens becomes a primary goal in the art.

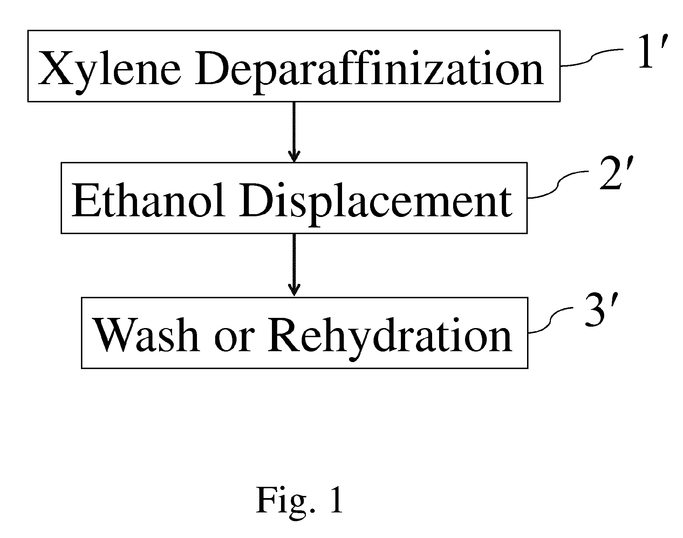

Therefore, prior to the steps of extracting nucleic acids, the first procedure is removing the embedding material (paraffin) to obtain the biological tissue, and this procedure is generally named "deparaffinization". However, most of the presently available methods for processing FFPE specimens are based on the conventional way of using organic solvents to remove the paraffin (refer to Fig. 1 showing the conventional deparaffinization procedure). The steps of this procedure are briefly described below.

### Step 1' Xylene Deparaffinization:

Pure xylene or xylene-based solutions with serial concentrations (generally from 70% to 100% v/v) is used to immerse the FFPE sample, and then the used xylene solvent/solution is displaced by fresh solvent/solution; and this process is repeated for a few times to completely remove the paraffin from the biological tissue. When the xylene-based solutions with serial concentrations are chosen for the aforementioned process, the xylene-based solutions should be used sequentially according to the concentrations, that is, high concentration xylene-based solution is used prior to low concentration ones to completely remove the paraffin. Then, the biological tissue embedded in the paraffin can be obtained.

### Step 2' Solvent (Ethanol) Displacement:

Ethanol (100% v/v) is used to immerse the biological tissue, and the frequency of this immersing process can be adjusted if necessary to thoroughly displace the xylene-based solution.

### Step 3' Washing or Rehydration:

Pure ethanol or ethanol-based solutions with serial concentrations (generally from 70% to 100% v/v, prepared by pure water) can be used to immerse the biological tissue to further remove the residual xylene solution. In other words, this process can be accomplished by using pure ethanol or by using ethanol-based solutions (high concentration to low concentration) for several times for washing out any residual xylene solution, to provide the biological tissue for following nucleic acid extraction and the subsequent analyses of nucleic acids. Additionally, the deparaffinized biological tissue can be deposited in aqueous solution for following usage depending on the user's demands.

According to the aforementioned conventional method, it involves multiple steps of solution displacement and wash while the deparaffinized tissue fractions are dispersed in the solution. Therefore, in order to transfer the solution for displacement or wash, the process of centrifugation is always required to gather the dispersed tissue fractions to the bottom of centrifuge tube to remove the used solution. Accordingly, the frequent solution transferring steps involved in the process of solution displacement and centrifugation often result in the risk of losing tissue samples, which is unfavorable for the integrity and efficiency of nucleic acid isolation. Furthermore, FFPE specimens are usually rare and the tissue samples are embedded in small amount, the unfavorable effects as previously described are even more manifest.

Moreover, regarding the downstream analysis of nucleic acids, the pretreatment procedure of conventional deparaffinization method prior to the nucleic extraction procedure can determine the quality of tissue samples, or inappropriate operation may even amplifies the unfavorable effects. For example, the residual paraffin and organic deparaffinizing solvent (xylene) are likely detrimental to the nucleic acid extraction efficiency and the accuracy of quality control. Consequently, the overall performance of extraction including the yield, the concentration, and the purity of nucleic acids may be deteriorated.

In view of the foregoing conventional method, in order to isolate nucleic acids from FFPE samples, it requires complicated and time-consuming process. On the other hand, toxic reagents are used which brings about the risk of safety and environmental problems. Moreover, as the diagnostic approaches based on genetic information develop vigorously, the quality of nucleic acids is more strictly required. In the meantime, as the demands of automatic facilities for diagnosis are increased, the high efficiency of isolating nucleic acids is highly demanded, and the efficient "high-throughput" goal is particularly demanded to accelerate the screening or diagnosis. Hence, improving the efficiency of isolating nucleic acids becomes a primary goal in the art.

The US patent application (publication number: US20070026432) proposes a method to isolate nucleic acids from FFPE specimens quickly for downstream analysis. However, according to this method, the paraffin is not practically dissolved out or separated from the tissue sample. Because the paraffin solidifies around its melting point (it is generally known that this phenomenon usually shows before the temperature rises at 60°C), the sample becomes aggregated, gelled, curded or even harden to be paraffin blocks. Therefore, the proposed method is quite sensitive to the temperature; in other words, the temperature must be kept constantly above the melting temperature to prevent the liquid paraffin to solidify; otherwise, the solidified paraffin will form aggregated precipitation or dispersion of gelled particles when the temperature drops, and consequently disturbs the follow-up procedures. Particularly, once the proposed method of nucleic acid extraction is further applied to automatic facilities, the foregoing problems may be even worse, because the automatic facilities for isolating nucleic acids are usually devised with complicated piping system and liquid transferring members. More specifically, the automatic facilities demand more qualified reagents or solutions to ensure that the pipe networks work properly. Regarding the unfavorable solidification of paraffin, the resultant non-liquid partials and gel are likely to obstruct the piping system to disturb the operation of automatic facilities accordingly, or even to wear down the automatic facilities. Moreover, the product quality and purification efficiency are also unfavorably affected, so that additional procedures (e.g., centrifugation and filtration) and facilities are required for solving the problems. Hence, the proposed method is not ideal for being applied to automatic facilities.

In summary, a working approach is currently demanded for simplifying the pretreatment procedures for FFPE specimens and achieving high-quality purification of nucleic acids. More importantly, this working approach should be easily applied to the current automatic systems without extra efforts or cost.

### SUMMARY OF THE INVENTION

Regarding the aforesaid problems of the conventional method for isolating nucleic acids from FFPE specimens, the present invention provides a method for isolating nucleic acids from a FFPE tissue sample containing a paraffin material and a biological tissue, comprising the steps of: (1) adding a first reagent and a second reagent to the FFPE tissue sample, the first reagent dissolving the paraffin material and the second reagent lysing the biological tissue; mixing the first reagent, the second reagent and the formalin-fixed paraffin embedded tissue sample to form a first mixture; (2) heating the first mixture at 50-80°C for 30-90 minutes; and then heating the first mixture at 80-95°C for 30-90 minutes to fractionize the first mixture to form an aqueous phase and an oil phase; (3) collecting an aqueous solution from the aqueous phase; and (4) isolating nucleic acids from the aqueous solution.

According to the method of this invention, the way of isolating nucleic acids from the aqueous solution includes: organic solvent extraction, affinity chromatography, and magnetic separation.

According to the method of this invention, wherein the first reagent is an oily reagent to dissolve the paraffin material in the FFPE tissue sample, and this oily reagent is selected from the group consisting of xylene, 6-bromohexyl acetate, citrosol and combinations thereof; and wherein the second reagent is an aqueous reagent to lyse the biological tissue, and this aqueous reagent contains at least one buffer, at least one surfactant and at least one metal chelating agent. This aqueous reagent further includes a protease.

According to the spirit of the invention, an objective of the invention is to provide a method for isolating nucleic acids from a FFPE tissue sample which can be generally applied to various reagents for deparaffinization. Furthermore, according to the method, the paraffin in the FFPE tissue sample are dissolved sufficiently and the choatropic solution are used simultaneously to lyse the biological tissue sample for the following procedures of extracting nucleic acids. Hence, the recovery of nucleic acids is improved to achieve the goal of simplifying the nucleic acids isolation process and thus promote the overall efficiency. Moreover, the method of the invention is particularly advantageous to small-size FFPE tissue samples.

Another objective of the invention is to provide a method for isolating nucleic acids from a FFPE tissue sample, which adds an oily reagent (deparaffinizing reagent) an aqueous reagent (choatropic reagent) to the FFPE tissue samples to prepare a mixture; wherein the paraffin embedding medium is dissolved into an oil phase and the biological tissue sample is lysed into an aqueous phase. Therefore, the aqueous phase can be easily used for extracting nucleic acids. Accordingly, the recovery of nucleic acids from the biological tissue and the nucleic acid extraction efficiency are improved; meanwhile, the problem of residual paraffin is prevented.

Still another objective of the invention is to provide a method for isolating nucleic acids from a FFPE tissue sample, which simplifies the pretreatment procedure of FFPE tissue samples without adopting manifold centrifugation steps, and improves the nucleic acid extraction efficiency; this method is extensively suitable for the current nucleic acid extracting methods, and this method also provides an ideal pretreatment process which can be simply applied to the current automatic devices/facilities and operation protocols thereof.

### BRIEF DESCRIPTION OF THE DRAWINGS

The present invention will be apparent to those skilled in the art by reading the following detailed description of a preferred embodiment thereof with reference to the drawings, in which:
Fig. 1 shows a diagram of the conventional deparaffinization process;
Fig. 2A shows a diagram illustrating the step S1 and S2 of the method for isolating nucleic acids from a formalin-fixed paraffin embedded tissue sample according to the invention;
Fig. 2B shows a diagram illustrating the step S3 and S3' of the method for isolating nucleic acids from a formalin-fixed paraffin embedded tissue sample according to the invention;
Fig. 3A shows the results of isolating DNA according to the Comparative Example 1 and the Examples 2-4 of the invention, which reflect the efficiency of isolating DNA;
Fig. 3B shows the results of isolating DNA according to the Comparative Example 1 and the Examples 2-4 of the invention, which reflect the effects of different biological tissue volumes on the efficiency of isolating DNA;
Fig. 4 shows the results of isolating DNA according to the Examples 5 and 6 and the Comparative Examples 6-8 of the invention, which reflect the effects of different biological tissue volumes on the efficiency of isolating DNA.
Fig. 5 shows the results of isolating DNA according to the Example 7 and the Comparative Example 8 of the invention; wherein the images demonstrate the appearance of the comparative samples of the Comparative Example 8 and the appearance of the aqueous solution of the Example 7.

### DETAILED DESCRIPTION OF THE PREFERRED EMBODIMENT

Certain example embodiments of this invention will now be described in more detail. The present invention relates to a method for isolating nucleic acids from FFPE tissue samples. Since the general technical details of processing FFPE specimens, isolation of nucleic acids from biological tissues, nucleic acid extraction, and the operation of device/apparatus thereof are known for those skilled persons in the art, the related techniques are not described in detail herein.

The method of the invention takes advantage of the chemical/physical properties of oily liquid (reagent) and aqueous liquid (reagent) as well as the interactions between those types of liquid. Practically, according to the method of the invention, the FFPE specimen/sample is firstly liquefied by dissolving the paraffin embedding medium allowing the formation of homogenous mixture. Then, the mixture is fractionized to form an aqueous phase and an oil phase, wherein the user can extract nucleic acids from the aqueous solution acquired from the aqueous phase. In comparison with the conventional complicated deparaffinization method as shown in Fig. 1, the method of the invention provides the more efficient and simple method which is also applicable for automatic operation. The following description details the advantageous technical features of the invention which is also described in certain embodiments.

The "oily reagent" described herein generally means the lipophilic liquid reagent or solution; and the "aqueous reagent" described herein generally means hydrophilic liquid reagent or solution. It is known that oily reagent and aqueous reagent are immiscible and that mixing the oily reagent and aqueous reagent usually forms two-phase liquid systems or colloids. The immiscible properties of the two liquid phases have been applied to separate hydrophilic molecules and hydrophobic molecules. However, the separation processes based on the foregoing properties usually involve complicated procedures of mixing oily reagent and aqueous reagent as well as manifold displacement of oily reagent or aqueous reagent.

On the basis of the aforementioned principles, regarding the FFPE specimens, they derive from living tissues (e.g., human tissue) which is abundant of aqueous solution; and thus, it is common that the biological tissues are treated by a process called fixation including the steps of fixing the tissues by fixers (e.g., formalin) immediately after sampling. Then a follow-up process called dehydration has to be performed to completely remove the moisture content in the tissues. Finally, the hydrophobic embedding medium such as paraffin is used to embed the tissues for long-term preservation of the tissue specimens, and the resultant specimens are the FFPE specimens/samples. The details of FFPE specimen preparation are known for the skilled persons in the art and not described herein. To concisely describe the practical features of the invention, the term of "FFPE tissue sample" hereinafter represents all the tissue sample and specimen embedded in paraffin embedding medium unless specified.

On the other hand, the FFPE tissue sample of the present invention generally means the fixed biological tissue embedded in paraffin, wherein the fixer includes formaldehyde or other formaldehyde-derived fixers. In other words, the invention does not limit the fixation methods or fixers for preparing FFPE sample even though the term of formalin is used to name the samples for the following reasons. Formalin generally means the aqueous solutions of formaldehyde; and the content of formaldehyde ranges from about 35-40 wt. %, and the 37 wt. % formaldehyde aqueous solution is the most widely used formalin. The fixer used for fixing the tissues is usually the neutralized 10% formalin solution, which means the fixer is prepared by diluting the formalin into a 10 v/v % formalin in an aqueous buffer system (formalin: buffer = 1: 9) to be the fixer with the formaldehyde content of about 4.1-4.5%. Furthermore, the user can add other additive(s) into the fixer as desired to optimize the fixation, for example, various buffers, acidic or alkaline reagents (for adjusting pH value) or methanol (generally in the content of 10 to 15 v/v % as an stabilizer for preventing polymerization) etc., can be optionally added. Therefore, the fixed specimens prepared by aforesaid fixers of formaldehyde, such as paraformaldehyde, are all referred to the FPPE tissue samples herein. Likewise, the invention does not limit the paraffin material to a specific type of paraffin. Regardless of the types of paraffin embedding medium or the embedding procedures is adopted for preparing the FFPE tissue sample, the method of the invention is widely applicable for broad types of FFPE tissue sample.

According to the forgoing technical principle of preparing FFPE specimens, in order to obtain the embedded biological tissue for analysis, the embedding paraffin material is the primary problem to be solved. Aiming at this problem, the conventional deparaffinization method (refer to Fig. 1) utilizes organic solvents, such as toluene and xylene, as the solution to remove the paraffin material by dissolving the paraffin material and then performing the complicated displacement procedures for removing embedding medium thoroughly. Then, based on aforementioned immiscible oil/aqueous two-phase system, the alcoholic reagents (such as ethanol and isopropanol) are further used for serial reagent displacement procedures called "washing processes". The washing processes are required to wash off the solvent. Alternatively, after removing the solvent, the solution can be optionally replaced by aqueous solution which is called "rehydration". Then the biological tissue without embedding medium is eventually used for nucleic acid extraction. More importantly, as the tedious pretreatment process proceeds, the biological tissue is likely to lose during the complicated and tedious procedures and thus disadvantageously affect the recovery of nucleic acids.

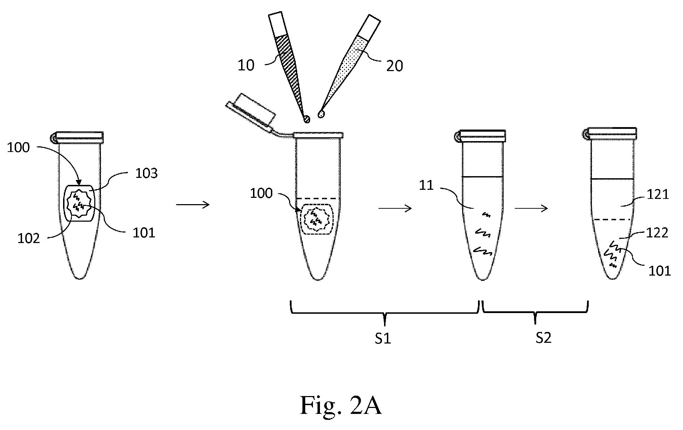

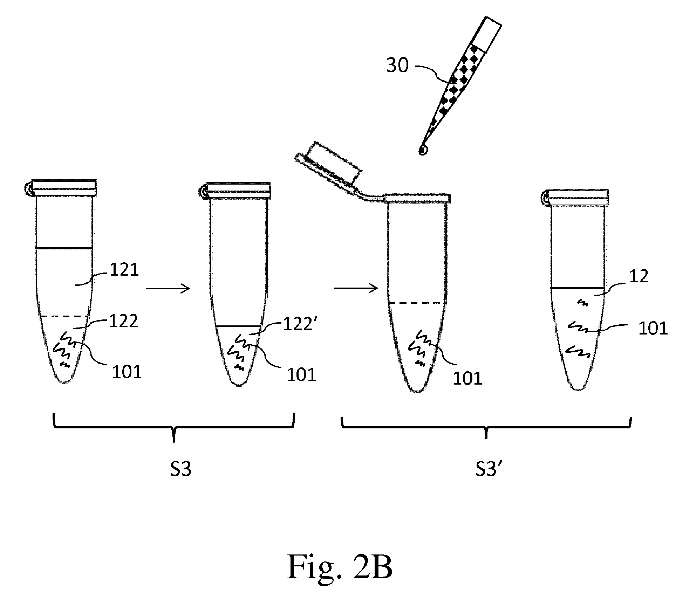

To solve the foregoing defects of the conventional art, the invention provides a method for isolating nucleic acids from FFPE tissue samples to easily isolate nucleic acids from a FFPE specimen. Refer to Fig. 2A and 2B illustrating the steps of the method of an embodiment according to the invention, as well as to the following description to demonstrate the method for isolating nucleic acids from the FFPE specimen according to the invention.

FFPE tissue sample 100 includes a paraffin material 103 and a biological tissue 102 which is the target tissue containing the nucleic acids 101. The nucleic acids 101 isolated according to the method of the invention are further applied to be further purified or analyzed. The present invention does not limit the application of the isolated nucleic acids 101, and the isolated nucleic acids 101 can be widely applied to all kinds of molecular analysis or immunological assays such as polymerase chain reaction (PCR), reverse transcription-PCR (RT-PCR), quantitative real time-PCR (Q-RT-PCR), microarray, immuno-precipitation, and so on.

Regarding the sample size (volume) of FFPE tissue sample 100, taking the pathological tissue for example, the sample size usually depends on the pathological case, and the factors affecting the washing processes include: the difficulty of sampling, the pathological lesion pattern (e.g., inflammatory site or non-inflammatory site) and the tissue type of the sampling tissue (e.g., epithelial tissue, connective tissue, muscle tissue and other unspecified sites). In general, the target sample tissues are usually rare and in small amount; or the tissues volume is restricted by sampling method (e.g., needle sampling). Consequently, the sample size is usually small and thus the nucleic acids are in small amount. Concerning this small sample size, the method of the invention is particularly advantageous to the tissue sample of small size, and the advantageous effects can be reflected by the following embodiments. Accordingly, the invention does not limit the sample size of biological tissue 102; in other words, the method is not only suitable for biological tissue 102 of normal size but also for the biological tissue 102 of small size.

The sample sizes of FFPE tissue sample 100 including large size, medium size, and small size, which are categorized in accordance with the volume of individual FFPE tissue sample 100. Practically, the volume of FFPE tissue sample 100 typically depends on the specific plane area and thickness of the individual FFPE tissue sample 100. The FFPE tissue sample 100 is usually obtained by sectioning from the whole block of paraffin specimen, thus FFPE tissue sample 100 is usually the paraffin-embedded tissue sections. Accordingly, based on that the general sizes of the tissue specimen block are not significantly different and the thickness of tissue sectioning is generally about 5-20 µm (0.005-0.02 mm), unless otherwise specified, the sample size of FFPE tissue sample 100 is substantially defined herein according to the plane area of the section. Hence, the large FFPE tissue sample is defined as having the plane area of 2.5-4 mm², the thickness of 5-20 µm and the volume of 0.0125-0.08 mm³. The medium FFPE tissue sample is defined as having the plane area of 1.5-2.5 mm² and the thickness of 5-20 µm and the volume of 0.0075-0.050 mm³. The small FFPE tissue sample is defined as having the plane area of 0.2-1.5 mm² and the thickness of 5-20 µm and the volume of 0.001-0.03 mm³. However, it is should be noted that, to those skilled persons in the art, it is known that the various methods an approaches may be utilized to acquire tissue samples clinically; therefore, the aforementioned definitions of sample size according to the plane area or volumes are merely specified to provide comprehensive embodiments, features, and advantages of the invention. Those definitions regarding the sample size are not intended to be used to limit the scope of the present invention. In other words, the present invention does not exclude any FFPE tissue sample of other size which is not covered by the aforementioned ranges (i.e., samples larger or smaller than the defined ones), since the unspecified cases may embody the present invention as well. More specifically, regarding the samples larger than the previously defined sample size, as long as the sufficient reagents are provided for essentially dissolving the whole FFPE tissue sample (i.e., within the processing capacity), the larger FFPE tissue samples are applicable for conducting the method of the present invention. On the other hand, regarding the samples smaller than the previously defined sample size, as long as the content or the recovery of nucleic acids are estimated to be sufficient to fulfill the user demands, which also means as long as the amount of nucleic acids is within the sensitivity range of the facility of detecting nucleic acids, the smaller FFPE tissue samples are applicable for conducting the method of the present invention. Therefore, in view of above, the FFPE tissue sample of different sizes can be practically applied to the method in accordance with the present application.

Firstly, referring to Fig. 2A showing the step S1 and S2 of the present invention. An FFPE tissue sample 100 is deposited in a microcentrifuge tube (or alternative container having the same function), and the step S1 is carried out by adding oily reagent 10 (meaning the first reagent) and aqueous reagent 20 (meaning the second reagent) to the previously deposited FFPE tissue sample 100 and mixing them well. During the process of mixing, because the oily reagent 10 is capable of dissolving paraffin material 103, while the aqueous reagent 20 is capable of lysing the biological tissue 102, the FFPE tissue sample 100 starts to liquefy after being mixed with the oily reagent 10 and aqueous reagent 20. The oily reagent 10, the aqueous reagent 20, and the liquefied biological tissue sample 100 are blended well to form a first mixture 11, wherein the volume ratio of the oily reagent 10 to the aqueous reagent 20 ranges from 0.5 to 4, which preferably ranges from 1 to 3, and more preferably ranges from 1.5 to 2.5.

The oily reagent 10 used in aforementioned steps are used for dissolving the paraffin material 103, and the oily reagent 10 according to the present invention generally includes the benzene organic solvents and the current deparaffinizaiton solvents; as long as the solvent is capable of dissolving paraffin, the solvent is applicable for the method according to the present invention. Specifically, the oily reagent 10 includes straight-chain or branched-chain hydrocarbons having 7-10 carbon atoms with or without benzene groups, which is in liquid form at ordinary temperature to be used as the solvent. It is not limited that the oily regent 10 to be single hydrocarbon (solvent) or a mixture solution of multiple hydrocarbons (solvents) in accordance with the present; on condition that the oily regent 10 is capable of dissolving paraffin, the oily regent 10 can be used as a proper solvent for deparaffinization. Preferably, the oily regent 10 includes toluene, xylene, 6-bromohexyl acetate, citrosol (CAS 65996-99-8) and combinations thereof. In other words, the oily reagent 10 is required to have ideal dispersion property in the aqueous reagent 20 which allows the oily reagent 10 to coexist with the aqueous reagent 20 and the oily reagent 10 can simultaneously dissolve the paraffin materials 103 sufficiently. In this way, the biological tissue 102 is exposed to the chaotropic aqueous reagent 20 sufficiently to promote the lysis of biological tissue 102 to release the nucleic acids 101 therein (as shown in Fig. 2A, the FFPE tissue sample 100 illustrated in dotted line).

As to the aqueous reagent 20, it is used for lysing the biological tissue 102 and it is not limited to be a specific type of aqueous reagent according to the invention, which means that the common aqueous choatropic reagents or commercial aqueous choatropic reagents for lysing biological tissue and cells are all suitable for the invention. Thus, the user can optionally select aqueous choatropic reagent of different constituents for specific tissue type. As long as the biological tissue 102 can be lysed well to expose the nucleic acids 101 to the aqueous reagent 20, the aqueous reagent 20 is applicable for the present invention. The aqueous reagent 20 includes a least one type of buffer (i.e., buffer system), at least one detergent, and at least one metal chelating agent (chelator). Preferably, the aqueous reagent 20 should include these constituents: buffer (e.g., Tris buffer system or high-salt buffer), chelating reagent for ions (e.g., EDTA), detergent (e.g., SDS, NP-40, Triton X-100, Tween-20, etc.), and protease (e.g., Proteinase K); and the proteinase inhibitors, inhibitors of specific purpose, and protein denaturing reagents (e.g., guanidine hydrochloride, GuHCl) can be optionally used simultaneously as demanded by the user to improve the tissue lysis.

It should be noted that, the oily reagent 10 and the aqueous reagent 20 are provided with ideal dispersion properties, and therefore, the invention does not limit the practical sequence of adding the oily reagent 10 and the aqueous reagent 20. Specifically, in addition to adding the two reagents sequentially, the oily reagent 10 and the aqueous reagent 20 can be blended or mixed prior to adding to the samples (as shown in Fig. 2A, the dotted line illustrated in step S1 represents the surface of a body of liquid before adding the reagents to the FFPE tissue sample 100). Practically, the oily reagent 10 and the aqueous reagent 20 are mixed sufficiently to ensure that the oily reagent 10 and the aqueous reagent 20 are mixed well with the dissolving FFPE tissue sample 100, and the purpose of the step S1 of the method is accomplished to obtain the first mixture 11.

Then, the step S2 firstly provides a heating process (not shown in Fig. 2A) to treat the mixture 11. The heating process is preferably divided into two phase with variant ranges of heating temperature. The first phase is to execute a heating temperature range of 50 to 80°C, preferably within the range of 50 to 65°C, and more preferably within the range of 53 to 60°C. The second phase is to execute a heating temperature range of 80 to 95°C, preferably within the range of 80 to 95°C, and more preferably within the range of 85 to 93°C. The duration of the aforementioned the first phase of heating process depends on the sample volume of FFPE tissue sample 100; in other words, the larger volume takes longer time for heating. According to the properties of FFPE tissue sample 100, if the volume of FFPE tissue sample 100 is large, the time duration can be prolonged to be an overnight heating to increase the dissolving of FFPE tissue sample 100. However, practically considering the working efficiency, the commonly used time duration ranges from 30 to 90 minutes; preferably ranges from 45 to 80 minutes; and more preferably ranges from 50 to 70 minutes. The time duration for the second heating phase ranges from 30 to 90 minutes; preferably ranges from 45 to 80 minutes; and more preferably ranges from 50 to 70 minutes.

According to the common knowledge of those skilled persons in the art, the heating method of step S2 is not limited practically. The heating process can be achieved by using a heater (or other heating device capable of heating the samples) to arrive at the predetermined temperature and then by depositing the microcentrifuge tube accommodating the first mixture 11 in the heater. Thus the first mixture 11 is heated to the predetermined temperature for the predetermined time duration. Besides, the first mixture 11 can be optionally deposited in the heater and then be heated to arrive at the predetermined temperature to accomplish the time duration of heating process. The purpose of heating process includes: enabling the materials, such as paraffin material 103 and biological tissue 102, in the first mixture 11 to dissolve or to lyse completely to release the nucleic acids 101 of the biological tissue 102 into the first mixture 11 for the follow-up application of extraction or purification. Practically, the first mixture 11 is preferably homogenized to indicate that the paraffin material 103 and the biological tissue 102 in the first mixture are dissolved and lysed sufficiently to release into the first mixture 11. These heating processes improve the paraffin material 103 to dissolve and promote the lysis of biological tissue 102, and thus promote the overall efficiency and quality of deparaffinization and tissue lysis. Additionally, it should be emphasized that the method of the invention advantageously avoids the complicated procedures of deparaffinization. Furthermore, the heating process can be executed after adding the oily reagent 10 and aqueous reagent 20, and thus the whole mixture are heated to achieve the effect of promoting deparaffinization and tissue lysis simultaneously, which is advantageously distinct from the conventional method that requires a heating process during the deparaffinziaiton to dissolve the paraffin and then further requires an additional heating process to lyse the tissue after accomplishing the deparaffinization. In comparison with the conventional method, the method of the invention apparently provides a simpler and more effective way of treating the FFPE tissue sample.

After accomplishing the previous steps, the paraffin material 103 of the FFPE tissue sample 100 has been solubilized in the oily reagent 10 which allows the biological tissue 102 to be sufficiently exposed to the aqueous reagent 20; meanwhile the aqueous reagent 20 can lyse the tissue/cell of the biological tissue 102 to release the nucleic acids into the first mixture 11. Another advantage of the invention is that the oily reagent 10 and the aqueous reagent 20 are used at the same time, and the both reagent coexist to bring their function into full play without interfering the effect of tissue lysis of the biological tissue 102. Consequently, the method of the invention can provide the oily reagent 10 and aqueous reagent 29 simultaneously to remove the paraffin material as well as to lyse the biological tissue 102 such that the working procedures is simplified, and thus the better efficiency is achieved.

Proceeding with the following the steps by taking the heated microcentrifuge tube containing the first mixture 11 out of the heater and allow it to stand at room temperature for a while until the first mixture starts to fractionize to form a aqueous phase 122 and an oil phase 121 (as shown in Fig. 2A, the dotted line illustrated in step S2 represents the interface of the fractionized liquid). In other words, because the first mixture 11 substantially still includes the immiscible oily reagent 10 and aqueous reagent 20, the phenomenon of fractionation (oil phase and aqueous phase) can be seen after standing for a while. The upper fraction is the oil phase 121 which is primarily formed by the oily reagent 10 and the lower fraction is aqueous phase 122 which is primarily formed by the aqueous reagent 20. The volume ratio of the oil phase 121 to the aqueous phase 122 depends on the volume ratio of the oily reagent 10 to the aqueous reagent 20 loaded in the previously described step S1. After the fractionation of the oil phase and the aqueous phase, the nucleic acids 101 and the residual biological tissue 102 (only when the incomplete tissue lysis occurs) is contained in the aqueous phase 122; while the dissolving paraffin material 103 and the constituents of the solvent are separated into the oil phase 121. Therefore, the aqueous phase 122 will be processed in the following procedures of nucleic acid extraction and purification. To acquire the aqueous phase 122, the user can optionally chose removing the oil phase 121 to remain the aqueous phase 122 or drawing out the aqueous phase 122 directly for the following steps. Preferably, drawing out the aqueous phase 122 directly would be more advantageous in accordance with the method of the invention.

Accordingly, the present invention provides a simple method of acquiring the aqueous phase 122 containing nucleic acids 101 as previously described, allowing that the aqueous phase 122 can be used directly to proceed with the following steps of isolating nucleic acids. In contrast, according to the conventional method, the user has to displace the organic solvents and aqueous solutions by manifold steps. Moreover, the sample wash steps are necessary for successful displacement, such as washing the deparaffinized sample (by xylene) with ethanol repeatedly to completely displace xylene and wash off any residual xylenes, so as to carry on the following steps of tissue lysis and nucleic acid isolation. Therefore, complicated centrifugation steps are required. The method of the present invention requires no complicated centrifugation steps for deparaffinization and solvent displacement to pretreat the samples for isolating the nucleic acids 101, and it apparently that the pretreatment process is simplified. However, if the user demands to accelerate the fractionation of the first mixture 11, the centrifugation procedure can be still adopted optionally.

Besides, according to the method of the present invention, even when the temperature of the heated first mixture 11 gradually cooled down to melting temperature of paraffin (usually ranges from 60 to 65 °C) or the temperature below that melting temperature, the first mixture 11 maintains in the form of liquid without formation of gelled paraffin or other phenomenon of aggregation. By contrast, the conventional methods adopting the heating process to dissolve the paraffin are usually limited by the temperature; in other words, the temperature should be consistently kept (preferably higher than the melting temperature of paraffin) to maintain the paraffin material in the liquid form until the paraffin material is eventually removed from the biological tissue, and the operation temperature can be lowered to the room temperature thereafter; otherwise the cooled paraffin will gelled or solidified to interfere the results of deparaffinization and the nucleic acid isolation. Therefore, the present invention provides a method which successfully prevents the problem of gelled/solidified paraffin due to the lowering temperature during the process, and the method of the invention obviously overcomes the aforementioned defects.

Additionally, since the volume of the biological tissue 102 are usually small, the biological tissue 102 processed according to the conventional method becomes detrital or in small pieces scattered in the solution, hence it is necessary to conduct centrifugation process to pull down and to gather the biological tissue 102 to the tip of the microcentrifuge tube for performing the previously described steps of solvent displacement or sample wash. However, even though the pieces of biological tissue 102 are gathered to from the pellet, the volume of the pellet is quite small relative to the volume of the solution (i.e., the solution to be removed). Therefore, a manual operation for removing the solution is particularly required for the following procedures such that it takes longer time and more complicated manual operations. Moreover, once there is any inappropriate operation, it may result in the problem of insufficient displacement, residual solvent and even the loss of biological tissue 102, those which negatively affect the quality of nucleic acid isolation. Accordingly, the method of the present invention involves no essential step of centrifugation and makes the method to be particularly advantageously for being applied to the biological tissue 102 in small amount.

Continue to refer the Fig. 2B illustrating the step S3 following the step S2. The purpose of step S3 is to further process the aqueous phase 122 containing the nucleic acids 101 for the following procedure of isolating the nucleic acids. By using the pipette to assess the aqueous phase 122 of the fractionized first mixture 11, an aqueous solution 122' is drawn out from the aqueous phase 122.

The present invention provides a simplified method for isolating the nucleic acids form FFPE tissue samples without adopting centrifugation steps. Besides, the volume of the oil phase 121 and the volume of the aqueous phase 122 are about the same or quite similar, which is advantageous for separating the aqueous phase 122.

Specifically, in view of the previous descriptions, the volume ratio of the oily reagent 10 to the aqueous phase 20 substantially ranges from 0.5 to 4; in other words, those skilled persons can easily understand that after the first mixture 11 become fractionized, the volume ratio of the oil phase 121 to the aqueous phase 122 can be estimated and then predetermined to reduce the difference of the volume between the oil phase 121 and the aqueous phase 122. Therefore, regarding the manual operation, because the interface between the oil phase and the aqueous phase 122 are quite clear and the predetermined advantageous volume ratio of the two phases, the user can easily draw out the solution from the aqueous phase 122 manually and meanwhile obtain sufficient and complete nucleic acid for isolation. Moreover, due to that the density of the aqueous phase 122 is higher than that of the oil phase 121, the aqueous phase 122 is situated in the lower fraction of the fractionized liquid near the tip of the microcentrifuge tube which allows the user/operator to easily draw out the aqueous solution 122' from the aqueous phase 122. More particularly, with respect to the volume of the aqueous phase 122, the recovery of the aqueous solution 122' can achieve 80% (i.e., the volume of the aqueous solution122' obtained relative to the volume of the whole aqueous reagent 20). Furthermore, when an automatic operation is applied, the performance of recovery could be even better, which effectively improves the problem of tissue loss of the biological tissue 102 during the complicated pretreatment process.

The convenience of the previously described procedure is not only advantageous to the manual operation but also reduce the pipette errors of sampling the liquid. As to the application of automatic facilities, the predetermined volume ratio of two phases (the oily reagent 10 and the aqueous reagent 20) allows the user to estimate the volume of the aqueous phase 122 and accordingly determined the volume of the aqueous solution 122' to be obtained, and therefore it characterizes the method of the present invention as a quantitative method, and makes this method to be quite suitable for being applied to the automatic pipetting facilities. In other words, the user can evaluate and determine the volume ratio of the reagent of the two phases, as well as the volume of aqueous solution 122' (reflected as the interface of the fractionized liquid body) prior to operation. The method is applicable for the current automatic facilities to immediately practice a standardized process of nucleic acid isolation without additional purchase of device is required. More specifically, because the method of the present invention does not require the centrifugation procedure, the current automatic facility can be adopted for operate the procedure of adding reagents or removing reagents. Meanwhile, the immiscible properties of the two phases and the constant volume ratio of these two phases enable the user to estimate the volume of the aqueous phase 122 to set up the automatic device so as to acquire the consistent volume of the aqueous phase 122 promptly and constantly for the following procedures of nucleic acid extraction. Moreover, those automatic operations are particularly advantageous in achieving the follow-up quantitative analysis. In summary, the applicability of the method for the automatic system can further prevent the artificial errors, sample loss, or mistaken sampling contaminations derived from manual operations.

The aqueous solution 122' is further processed according to the step S3'. The purpose of step S3' includes (1) ensuring the residual, if present, can be further lysed to completely recover the nucleic acids 101; (2) executing the step S3' according to the chosen follow-up nucleic acid extraction method, and adjusting the constituents of the reagent to improve the overall performance of nucleic acid extraction. According to the step S3', a choatropic reagent 30 (i.e., the third reagent) capable of lysing the biological tissue 102 is added into the aqueous solution 122' and mixed well to form the second mixture 12. Since the choatropic reagent is primarily used for lysing the residual biological tissue 102, the constituents should fulfill the demand of "lysing the biological tissue and cells". Thus, the commonly used choatropic reagents or the commercial choatropic reagents for lysing the biological tissue and cells are all suitable for the method of the present invention. Depending on the type of biological tissue 102 or other demands, the user can flexibly decide the type of choatropic reagent for practicing the method as long as the choatropic reagent effectively lyses the biological tissue 102 to release the nucleic acids 101 into the second mixture 12. Besides, the constituents of the choatropic reagent 30 can be modified according to the different nucleic acid extraction approaches, such as organic solvent extraction, magnetic separation and affinity chromatography to optimize the resultant second mixture 12 into the condition suitable for being applied to the follow-up procedures of nucleic acid extraction. For example, when the affinity chromatography is preferred, the guanidinium chloride (GuCl) or the guanidine hydrochloride (GuHCl) is optionally added to the choatropic reagent 30 to allow the choatropic reagent 30 to enable the second mixture 12 to completely lyse the residual proteins, and thus the second mixture 12 containing the nucleic acids 101 provides the affinity membrane (filter) an optimal electric charge properties to promote the binding affinity between the nucleic acids 101 and the membrane (filter) such that the overall performance of nucleic acid extraction is improved. According to a preferred embodiment of the present invention, the choatropic reagent 30 usually includes the at least one buffer and at least one detergent. The choatropic reagent 30 preferably contains those listed constituents: the buffer (e.g., Tris buffer system), multiple detergents (e.g., SDS, NP-40, Triton X-100, and Tween-20, etc.), and denaturing reagent (e.g., urea and guanidinium chloride, etc.); and various enzyme inhibitors can be optionally added to adjust the constituents of the choatropic reagent 30 as demanded by the user. Therefore, the present invention does not limit the constituents and composition of the choatropic reagent 30.

The step S4 (not illustrated in the drawing) is further executed to extract nucleic acids 101 from the mixture 12. According to the previously described steps S1 to S3, the second mixture 12 is obtained, and the step S3' is optionally conducted to process the second mixture 12. The second mixture 12 containing nucleic acids 101 requires further extraction and purification procedures to obtain the nucleic acids 101 from the second mixture 12. The present invention does not limit the way of extracting and purifying the nucleic acids 101. In other words, the concepts and embodiments according to the present invention generally cover the current approaches of nucleic acid extraction and purification. Nonetheless, the widely used current approaches of nucleic acid extraction and purification include: organic solvent extraction, affinity chromatography, and magnetic separation. Regarding the organic solvent extraction, the phenol-chloroform method is the most common. Regarding the affinity chromatography method, the spin-column based method is the most common. Regarding the magnetic separation method, the magnetic beads adsorption, which has been generally applied to the automatic facilities, is the most common.

Since the principles, technical details of aforementioned nucleic acid extraction approaches are known for those skilled persons in the art, and the approaches respectively include advantages or drawbacks, thus it is not described in detail herein. Some brief examples are listed as follow. Utilizing the organic solvent extraction method may acquire nucleic acids in high purity and quality; while this method is more likely to loss sample and bring about the safety concerns of organic solvents. Utilizing the affinity chromatography method may be prompt and convenient, while it is usually more expensive and it requires the centrifuge device and various specific formulated reagents to fully achieve the expected extraction effects. As to the magnetic separation method, it is a simple and safe method, while it is more expensive than the organic extraction method. However, the magnetic separation method achieves an excellent extraction performance with simple and safe operations, and it is quite suitable for being applied to the automatic systems to conduct the high throughput analysis.

In view of above, the present invention provides a simplified method for isolating nucleic acids from a FFPE tissue sample which integrates the process of deparaffinization and the process of biological tissue lysis to remove the paraffin material and lyse the biological tissue simultaneously. In this way, the problem of tissue loss derived from complicated processing procedures can be effectively prevented. Thus, the nucleic acid isolation process of ideal quality and recovery (yield) is accomplished more efficiently according to the method of the present invention.

Some examples and comparative examples according to the preferred embodiments of the present invention are recited herein to fully disclose the spirit and embodiment of the present invention to enable those skilled persons in the art can easily practice the method of the invention.

First of all, all the following description concerning the FFPE tissue sample sizes are characterized into three groups in light of the volume of FFPE tissue samples: small FFPE tissue samples, medium FFPE tissue samples and large FFPE tissue samples; and the practical size ranges of those FFPE tissue samples are well-defined in the previous description and are not described again.

### Comparative Example 1

### Conventional deparaffinization method by xylene (Conventional pretreatment)

In this comparative example, a conventional pretreatment of deparaffinization by xylene is used to pretreat the small, medium and large FFPE tissue samples for removing the paraffin respectively, followed by the "affinity chromatography method" to obtain the nucleic acid products. The steps are described as follow.

Add 1 ml xylene to the microcentrifuge tubes (hereinafter "microtubes") accommodating the small, medium and large FFPE tissue samples respectively and mix those materials by vortex. Let the microtubes to stand at the temperature of 60°C for 10 min. Then centrifuge (12,000 rpm) the microtubes containing those materials for 10 min at room temperature and take out the microtubes to remove the supernatant. Add 1 ml 95% ethanol (EtOH) into the microtubes, mix by vortex, and let the microtubes stands for 10 min at room temperature. Centrifuge the microtubes (12,000 rpm) at room temperature for 10 min at room temperature and take out the microtubes to remove the supernatant. Dry the pellet at 60°C for 10 min. Add 180µl choatropic reagent A (1% SDS, 30 mM Tris-HCl, 10 mM EDTA) and 20µl proteinase K into the microtubes and heat the whole mixture at 56°C for 1 hour, and let the microtubes stand at room temperature for 1 hour. Meanwhile, start the heater (or the heating device) to arrive at 90°C. Put the microtubes of mixture into the heater and heating the mixture at 90°C for 1 hour followed by adding 200 µl choatropic reagent B (5% TritonX-100, 10% Tween-20, 5M GuHCl). Mix those materials well by vortex. Then, add 200 µl 100% EtOH and mix well. Spin down the whole mixture to obtain the sample mixture solution.

Then, extract the nucleic acids respectively from the aforesaid sample mixture solutions of the small, medium and large FFPE tissue samples by the QIAamp DNA FFPE Tissue Kit (QIAGEN; Cat. No. 56404). This kit provides spin column-based nucleic acid extraction and purification according to the affinity chromatography method. The kit is practically used according to the instruction of the manufacturer which is described briefly as follow.

Load all the sample mixture solution into the spin columns respectively, and centrifuge for 1 min (8,000 rpm); discard the used collection tubes and apply new collection tubes; add 500 µl Buffer W1 into the spin columns and centrifuge for 1 min (8,000 rpm); replace the used collection tubes with new ones; add 500 µl Buffer Wash into the spin columns and centrifuge for 1 min (8,000 rpm); replace the used collection tubes with new ones; centrifuge (13,000 rpm) the spin columns once more time for 3 min without adding any reagent. Then transfer the spin columns to new microtubes; add 60 µl pre-heated Elution Buffer onto the filter membranes of the spin columns respectively; and let stand for 5 min. Finally, centrifuge (13,000 rpm) the spin columns, together with the microtubes, for 1 min to obtain the nucleic acid products.

### Example 2

### Xylene-based pretreatment method of dissolving paraffin (Pretreatment 1)

The Example 2 describes one preferred embodiment according to the present invention, which describes an example of pretreating the small, medium and large FFPE tissue samples respectively for removing the paraffin and lysing the biological tissue samples simultaneously, followed by the "affinity chromatography method" to obtain the nucleic acid products. The steps are described as follow.

Firstly, add 400 µl xylene (oily reagent), 180 µl aqueous reagent (1% SDS, 30 mM Tris-HCl, 10 mM EDTA) and 20 µl proteinase K into the microtubes accommodating FFPE tissue samples respectively and mix those materials by vortex for 10 seconds to obtain a mixture 2A. Then heat the whole mixture 2A at 56°C for 1 hour, and let the microtube stand at room temperature for a while. Meanwhile, start the heater (or the heating device) to arrive at 90°C; and put the microtube containing the mixture 2A into the heater and heating the mixture at 90°C for 1 hour. Because the moisture is generated inside the microtube during the heating process, spin the microtube shortly to spin down the moisture back to the mixture 2A. At the same time, it should be observed that the mixture 2A starts to fractionize, wherein the upper phase is the oil phase and the lower phase is the aqueous phase. Then, acquire the aqueous solution (about 190 µl) from the lower aqueous phase by a pipette, and transfer the aqueous solution to new microtube. Add 200 µl choatropic reagent (5% TritonX-100, 10% Tween-2, 5M GuHCl) to the aqueous solution and mix well to obtain the mixture 2B.

Mix the mixture 2B uniformly, and then add 200 µl 100% EtOH to the mixture 2B and mix well. Spin down the whole aforesaid materials shortly to obtain the sample mixture solution.

Then, extract the nucleic acids respectively from the aforesaid sample mixture solutions of the small, medium and large FFPE tissue samples by the QIAamp DNA FFPE Tissue Kit (QIAGEN; Cat. No. 56404). However, this extraction procedure is substantially the same with the extraction procedure of the Comparative Example 1, and thus it is not described again.

### Example 3

### 6-bromohexyl acetate-based pretreatment method of dissolving paraffin (Pretreatment 2)

The Example 3 describes one preferred embodiment according to the present invention, which describes an example of pretreating the small, medium and large FFPE tissue samples respectively for removing the paraffin and lysing the biological tissue samples simultaneously, followed by the "affinity chromatography method" to obtain the nucleic acid products. The steps of practicing the Example 3 are substantially the same with the Example 2, with the only difference in that the oily reagent is 6-bromohexyl acetate in Example 3. Thus it is not described in detail.

### Example 4

### Citrosol-based pretreatment method of dissolving paraffin (Pretreatment 3)

The Example 4 describes one preferred embodiment according to the present invention, which describes an example of pretreating the small, medium and large FFPE tissue samples respectively for removing the paraffin and lysing the biological tissue samples simultaneously, followed by the "affinity chromatography method" to obtain the nucleic acid products. The steps of practicing the Example 4 are substantially the same with the Example 2, with the only difference in that the oily reagent is citrosol in Example 4. Thus it is not described in detail.

### Example 5

### Automatic magnetic separation of nucleic acids (Automatic magnetic separation method)

The Example 5 describes one preferred embodiment according to the present invention, which describes an example of pretreating the small, medium and large FFPE tissue samples respectively for removing the paraffin and lysing the biological tissue samples simultaneously, followed by the "automatic magnetic separation method" to obtain the nucleic acid products.

Concerning the procedure of pretreatment of the FFPE tissue samples, the steps are substantially the same with the Example 2, with the following differences: in the initial step, add 400 µl xylene (oily reagent) and 400 µl aqueous reagent (1% SDS, 30 mM Tris-HCl, 10 mM EDTA) and 20 µl proteinase K into the microtubes accommodating FFPE tissue samples respectively and process the samples as previously described in the Example 2 to obtain the mixture, and acquire the aqueous solution from the lower aqueous phase of the fractionized mixture; and in the nucleic acid extraction steps, the aqueous solution is applied to the automatic magnetic separation device (MagCore® HF-16; RBC bioscience) to perform the magnetic separation to ultimately obtain 60 µl nucleic acid product respectively from each FFPE tissue sample. Besides, if the automatic magnetic separation device is equipped with heating apparatus, the previous pretreatment steps can be conducted by the same automatic device.

### Comparative Example 6

### Manual method 1: extracting nucleic acids by affinity chromatography

The Comparative Example 6 describes the widely used conventional deparaffinization method of pretreating the small, medium and large FFPE tissue samples respectively to obtain the nucleic acid products. According to the Comparative Example 6, the kit for deparaffinization (QIAGEN; QIAamp DNA FFPE Tissue Kit; Cat. No. 56404) is used to remove the paraffin and extract the nucleic acids, and the operative procedures are based on the instruction of the manufacturer described briefly as follow.

Firstly, add 1 ml xylene to the microtubes accommodating the FFPE tissue samples respectively and mix those materials by vortex for 10 seconds. Centrifuge (13,000 rpm) the microtubes containing those materials for 2 min at room temperature. Then, draw the supernatant out of the microtube by pipette and discard the supernatant. During the pipetting process, the operator needs to pay attention to prevent disturbing or sucking out the pellet at the bottom of the microtube. Then, add 1 ml 95% EtOH into the microtube and wash off the residual xylene by vortex, followed by the centrifugation (13,000 rpm) for 2 min at room temperature. Remove the EtOH by pipette or suction from the microtube and open up the lid of the microtube to stand at room temperature or to heat the microtube at 37°C, such that the EtOH can be sufficiently vaporized. Likewise, the operator needs to pay attention to prevent disturbing or sucking out the pellet at the bottom of the microtube during the process of removing the liquid. Then, add 180µl Buffer ATL and 20µl proteinase K into the microtube and mix the Buffer ATL with the pellet by vortex, heat the whole mixture at 56°C for 1 hour, and let the microtube stand at room temperature for 1 hour.

Meanwhile, start the heater (or the heating device) to arrive at 90°C, heat the mixture at 90°C for 1 hour followed by spinning down the moisture generated during the heating process. Add 200 µl Buffer AL and mix well all the solutions. Then add 200 µl 100% EtOH to form a sample mixture solution which is further loaded into the spin column, and execute the affinity chromatography method by the QIAamp DNA FFPE Tissue Kit (QIAGEN; Cat. No. 56404) to obtain the nucleic acid products. The extraction procedure is substantially the same as previously described and thus it is not described again. The final step is the elution of the nucleic acid products by 60 µl Buffer ATE.

### Example 7

### Manual method 2: extracting nucleic acids by affinity chromatography

The Example 7 describes one preferred embodiment according to the present invention, which describes an example of pretreating the small, medium and large FFPE tissue samples respectively for removing the paraffin and lysing the biological tissue samples simultaneously, followed by the "affinity chromatography method" to obtain the nucleic acid products. The steps of practicing the Example 7 are substantially the same with the Example 2, with the only difference in that another nucleic extraction kit (RBC Bioscience, Genomic DNA Extraction Kit (Tissue), Cat. No. YGT) is used in Example 7, and the procedures are performed according to the manufacturer's instruction. In brief, load the aforesaid sample mixture solution into the spin column and wash by serial buffers and centrifugation, and ultimately elute the nucleic acid products by 60 µl elution buffer.

### Comparative Example 8

### Manual method 3: extracting nucleic acids by affinity chromatography

The Example 8 describes one preferred embodiment according to the present invention, which describes an example of pretreating the small, medium and large FFPE tissue samples respectively to lyse the biological tissue samples, followed by the "affinity chromatography method" to obtain the nucleic acid products. The steps of practicing the Example 8 are substantially the same with the Example 7, with the only difference in that the pretreatment method used prior to the affinity chromatography method. Specifically, regarding the Example 8, only aqueous reagent is used to pretreat the FFPE tissue samples immediately followed by the nucleic acid extraction. More particularly, in the Example 8, add 180 µl aqueous reagent (1% SDS, 30 mM Tris-HCl, 10 mM EDTA) and 20 µl proteinase K into the microtubes accommodating FFPE tissue samples respectively and mix by vortex. Heat the whole mixture at 56°C for 1 hour, and let the microtube stand at room temperature,

Meanwhile, start the heater (or the heating device) to arrive at 90°C, heat the mixture at 90°C for 1 hour followed by spinning down the moisture generated during the heating process. At the meantime, the paraffin materials start to solidify and the solution fractionizes into a liquid phase and a solid phase. Acquired 190 µl aqueous solution from the liquid phase and transfer the aqueous solution into a new microtube. Add 200 µl choatropic reagent (5% TritonX-100, 10% Tween-20, 5M GuHCl) into the microtube and mix well to obtain a mixture, then add 200 µl 100% EtOH, mix well and spin down the mixture. Load the mixture into the spin column and execute the affinity chromatography method by the nucleic extraction kit (RBC Bioscience, Genomic DNA Extraction Kit (Tissue), Cat. No. YGT) to obtain the nucleic acid products.

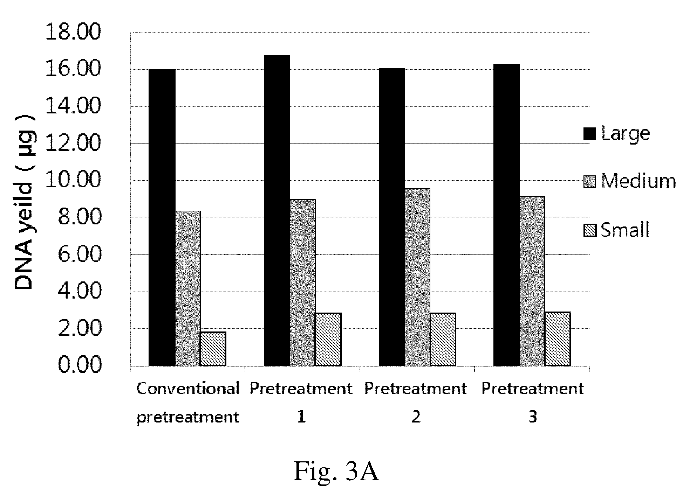

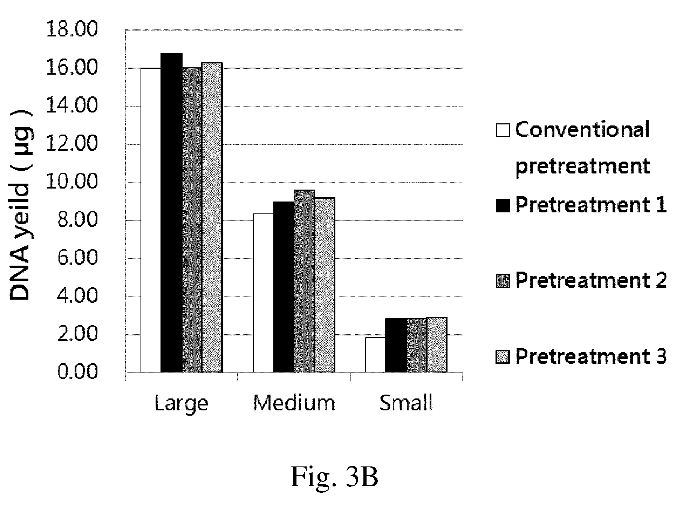

According to the forgoing Examples and Comparative Examples, the nucleic acid products are quantified or assayed to evaluate the yield of nucleic acids, and the advantages of the present invention will be described accordingly. First of all, refer the Fig 3A and 3B, which represent the comparative results of nucleic acid isolation efficiency concerning the previously described Comparative Example land Example 2-4. The comparative results reflect that the present invention provides a method that achieves approximately the same nucleic acid isolation efficiency as well as the nucleic acid recovery efficiency of the conventional method, for the small, medium and large FFPE tissue samples. Particularly, the present method is more advantageous to the small FFPE tissue samples. The results indicate that the method of the present invention is not only simple and convenient, but also excellent in the yielding performance. Besides, in view of the results of the widely used solvents including xylene, 6-bromohexyl acetate, and citrosol described in the previous examples, it is apparently proved that the method of the present invention is suitable for being applied to the mentioned solvent systems, which reasonably indicates that the method is widely applicable for current solvent systems of deparaffinization. Thus, the current solvent systems can be flexibly chosen to fulfill the demands of analysis, cost, and safety. Therefore, other solvents capable of dissolving paraffin materials are included in view of the scope of the present invention.

Particularly, with respect to Table 1 representing the results of calculated differences according to the Comparative Example 1 and Example 2-4, the data are calculated on the basis of the result of the nucleic acid yield (DNA yield) of the Comparative Example 1, wherein the DNA yield of the specific Example (Example 2-4) are divided by the DNA yield of the Comparative Example 1 (Conventional pretreatment) for normalization. Table 1 shows the comparative results of the normalized data. For example, the "Pretreatment 1/Conventional pretreatment" means the quotient of the DNA yield of Pretreatment 1 divided by the DNA yield of Conventional pretreatment.

**Table 1.**

| **Comparaitve analysis of DNA yeild of different nucleic acid isolation methods using different pretrea** | | | |
|---|---|---|---|
| | Large sample | Mediumsample | Small sample |
| Pretreatment 1 /Conventional pretreatment | 1.05 | 1.07 | 1.55 |
| Pretreatment 2 /Conventional pretreatment | 1.00 | 1.15 | 1.55 |
| Pretreatment 3/Conventional pretreatment | 1.02 | 1.10 | 1.57 |

Refer to Fig 3A and 3B and Table 1, it should be noted that, the DNA yield according to the method of the present invention reflects that this method becomes more advantageous as the volume of the FFPE tissue samples become smaller. More specifically, with respect to the results of the large FFPE tissue samples, the DNA yield of Pretreatment 1 (xylene-based) is slightly higher than that of Conventional pretreatment (xylene-based); with respect to the results of the medium FFPE tissue samples, the DNA yield of Pretreatment 1, as well as Pretreatment 2 and Pretreatment 3, show more superior effects than that of Conventional pretreatment; and, with respect to the results of the small FFPE tissue samples, the results show much more superior performance. And it indicates that the method of the present invention overcomes the previously described problems of tissue loss during the processing or washing steps as the tissue samples become smaller, which is common in the conventional method.

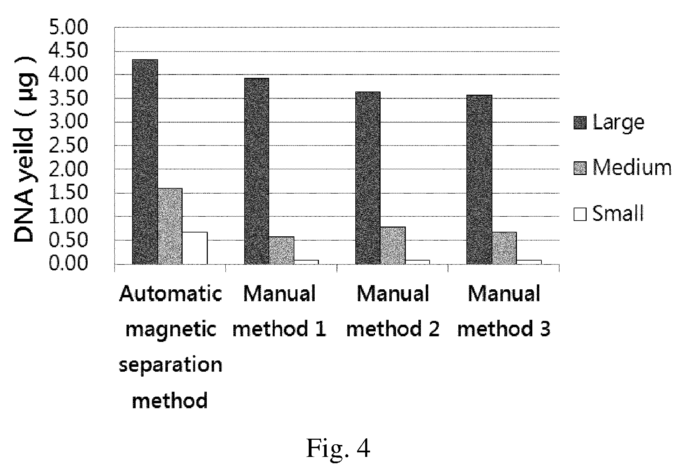

Refer to Fig. 4 representing the comparative results of nucleic acid isolation efficiency concerning the Examples 5, 7 and the Comparative Examples 6, 8 with different sizes (volume) of FFPE tissue samples. The comparative results recited in Fig. 4 show that the method of the present invention is not only suitable for automatic magnetic separation devices (Automatic magnetic separation method), but also applicable to the automatic magnetic separation of the small, medium and large FFPE tissue samples. Furthermore, the nucleic acid isolation efficiency is better than other methods of manual operation (Manual methods 1, 2, and 3). Besides, in comparison with the most widely used conventional deparaffinization method (Manual method 1 using the QIAGEN kit for conventional pretreatment and nucleic acid extraction), different reagent combinations (Manual method 3 using the kit manufactured by RBC Bioscience for conventional pretreatment and nucleic acid extraction) do not result in significant difference in the nucleic acid isolation efficiency. However, according to the method of the present invention (Manual method 2), the nucleic acid isolation efficiency is comparable to the conventional methods. It indicates that the method of the present invention indeed simplifies the procedures of processing FFPE tissue samples to improve the overall efficiency. It should be also noted that, when the method is practiced on the automatic system, the advantageous effect is more manifest.

Refer to Table 2 representing the results of calculated differences according to the specific Examples. The calculation is substantially the same as previously described regarding the result of Table 1, and it is not repeated. Table 2 shows that the method of the present invention (Manual method 2) significantly simplifies the complicated operative procedures, and achieve the comparable efficiency of the conventional method (Manual method 3). It is apparently that the present invention provides a method that overcomes the existing problems. Moreover, when the present invention is applied to the automatic magnetic separation system, the method is more advantageous than the recited manual operation methods (Manual method 1, 2 and 3), and the advantageous effects becomes more remarkable as the FFPE tissue sample becomes smaller (the DNA yield increases by more than 7 times over the large FFPE tissue samples). Therefore, the present method is particularly suitable for processing small FFPE tissue samples. In view of the above-identified advantages, the user can make full use of these features to the high-throughput nucleic acid isolation and the follow-up diagnostic screening to improve the overall effects of analysis.

**Table 2.**

| **Comparative analysis of DNA yeild of different nucleic acid isolation methods** | | | |
|---|---|---|---|
| | Large sample | Medium sample | Small sample |
| Automatic magnetic separation method/Manual method 1 | 1.11 | 2.79 | 7.35 |
| Automatic magnetic separation method/Manual method 2 | 1.19 | 2.04 | 7.52 |
| Automatic magnetic separation method/Manual method 3 | 1.21 | 2.34 | 7.86 |
| Manual method 2/Manual method 3 | 1.02 | 1.15 | 1.05 |

In addition to the comparison between the conventional method and the present invention, the Example 8 is based on the previously disclosed method and concept according to the US patent application (US20070026432) as comparison to verify the effects of nucleic acid isolation of the invention. To achieve the better comparative illustration, the mixture sample solution of Example 7 is further used for comparing the mixture sample solution of Example 8 which based on the disclosed method.

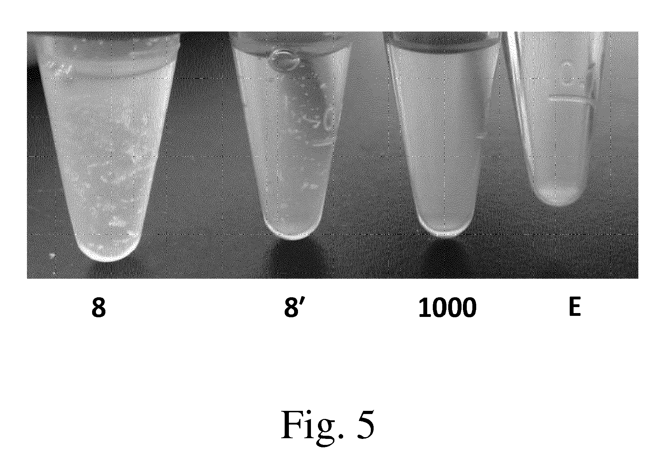

According to the comparative results, similar amounts of DNA yield is respectively obtained by the two methods, whereas the method of the present invention is more advantageous than the disclosed method of the Example 8 in overcoming the problem of precipitation and the resultant interference. Specifically, refer to Fig. 5 showing the images of sample solutions 8 and 8' which are both the mixture sample solution according to the Example 8, and also showing the image of the aqueous solution sample 1000 according to the Example 7. Empty microtube E is the empty microtube containing no samples. According to Fig. 5, it is apparently that the sample solutions prepared by the method of the Example 8 are turbid, and suspension and white precipitations are apparently observed. In contrast, the aqueous solution sample 1000 according to the Example 7 is clear and transparent.

In view of above, according to the method of the Example 8 even though nucleic acids can be sufficiently recovered, the problems of solidified paraffin such as turbid solution and precipitation interfering the operations apparently remain. Particularly, this previously disclosed method is disadvantageous for the automatic system and thus the applicability of the method is limited. In contrast, the method of the present invention provides a simple and convenient way to obtain clear aqueous solution, and when accompanied by the aforementioned advantages, the method for isolating nucleic acids from FFPE tissue sample of the present invention is quite suitable for being applied to the automatic system.

## Claims

1. A method for isolating nucleic acids from a formalin-fixed paraffin embedded tissue sample containing a paraffin material and a biological tissue; and the method is **characterized in that** the method comprises the following steps:
(1) adding a first reagent and a second reagent to the formalin-fixed paraffin embedded tissue sample, the first reagent dissolving the paraffin material and the second reagent lysing the biological tissue; mixing the first reagent, the second reagent, and the formalin-fixed paraffin embedded tissue sample to form a first mixture;
(2) heating the first mixture at 50-80°C for 30-90 minutes; and then heating the first mixture at 80-95°C for 30-90 minutes to fractionize the first mixture to form an aqueous phase and an oil phase;
(3) collecting an aqueous solution from the aqueous phase; and
(4) isolating nucleic acids from the aqueous solution.

2. The method of claim 1, wherein the first reagent is an oily reagent.

3. The method of claim 2, wherein the oily reagent containing straight-chain or branched-chain hydrocarbons having 7-10 carbon atoms.

4. The method of claim 2, wherein the oily reagent is selected from the group consisting of xylene, 6-bromohexyl acetate, citrosol and combinations thereof.

5. The method of claim 1, wherein the second reagent is an aqueous reagent containing at least one buffer, at least one detergent and at least one metal chelating agent.

6. The method of claim 5, wherein the aqueous reagent contains a protease.

7. A method for isolating nucleic acids from a formalin-fixed paraffin embedded tissue sample containing a paraffin material and a biological tissue; and the method is **characterized in that** the method comprises the following steps:
(1) adding a first reagent and a second reagent to the formalin-fixed paraffin embedded tissue sample, the first reagent dissolving the paraffin material and the second reagent lysing the biological tissue; mixing the first reagent, the second reagent, and the formalin-fixed paraffin embedded tissue sample to form a first mixture;
(2) heating the first mixture at 50-80°C for 30-90 minutes; and then heating the first mixture at 80-95°C for 30-90 minutes to fractionize the first mixture to form an aqueous phase and an oil phase;
(3) collecting an aqueous solution from the aqueous phase; and
(4) isolating nucleic acids from the aqueous solution by organic solvent extraction.

8. A method for isolating nucleic acids from a formalin-fixed paraffin embedded tissue sample containing a paraffin material and a biological tissue; and the method is **characterized in that** the method comprises the following steps:
(1) adding a first reagent and a second reagent to the formalin-fixed paraffin embedded tissue sample, the first reagent dissolving the paraffin material and the second reagent lysing the biological tissue; mixing the first reagent, the second reagent, and the formalin-fixed paraffin embedded tissue sample to form a first mixture;
(2) heating the first mixture at 50-80°C for 30-90 minutes; and then heating the first mixture at 80-95°C for 30-90 minutes to fractionize the first mixture to form an aqueous phase and an oil phase;
(3) collecting an aqueous solution from the aqueous phase; and
(4) isolating nucleic acids from the aqueous solution by affinity chromatography.

9. A method for isolating nucleic acids from a formalin-fixed paraffin embedded tissue sample containing a paraffin material and a biological tissue; and the method is **characterized in that** the method comprises the following steps:
(1) adding a first reagent and a second reagent to the formalin-fixed paraffin embedded tissue sample, the first reagent dissolving the paraffin material and the second reagent lysing the biological tissue; mixing the first reagent, the second reagent, and the formalin-fixed paraffin embedded tissue sample to form a first mixture;
(2) heating the first mixture at 50-80°C for 30-90 minutes; and then heating the first mixture at 80-95°C for 30-90 minutes to fractionize the first mixture to form an aqueous phase and an oil phase;
(3) collecting an aqueous solution from the aqueous phase; and
(4) isolating nucleic acids from the aqueous solution by magnetic separation.
